# EUROPÄISCHE PATENTSCHRIFT

(11) **EP 3 576 629 B1**
(45) Veröffentlichungstag und Bekanntmachung des Hinweises auf die Patenterteilung: **06.01.2021**
(21) Anmeldenummer: 18708045.2
(22) Anmeldetag: 01.02.2018
(51) Int. Cl.: A61B 6/02, A61B 6/00

(54) **COMPUTERTOMOGRAPH**
COMPUTER TOMOGRAPH
TOMODENSITOMÈTRE

(30) Priorität: 01.02.2017 DE 102017000994
(43) Veröffentlichungstag der Anmeldung: 11.12.2019
(73) Patentinhaber: Esspen GmbH, 91301 Forchheim (DE)
(72) Erfinder: MOHAMMADI, Zahra, 91052 Erlangen (DE)
(74) Vertreter: Meyer, Rudolf
(86) Internationale Anmeldenummer: PCT/EP2018/025024
(87) Internationale Veröffentlichungsnummer: WO 2018/141485

(56) Entgegenhaltungen:
- WO-A1-2009/012453
- WO-A2-2007/038306
- DE-A1-102010 062 541
- QIAN XIN ET AL: "Design and characterization of a spatially distributed multibeam field emission x-ray source for stationary digital breast tomosynthesis", MEDICAL PHYSICS, AIP, MELVILLE, NY, US, Bd. 36, Nr. 10, 4. September 2009 (2009-09-04), Seiten 4389-4399, XP012129710, ISSN: 0094-2405, DOI: 10.1118/1.3213520

## Beschreibung

Die Erfindung betrifft einen Computertomographen für die mammographische Röntgenbildgebung, bei welchem eine Rotation einer Röntgenröhre zur Röntgenbildgebung nicht erforderlich ist und welcher eine Multi-Fokus-Feldemissionsröntgenröhre aufweist, wobei die Multi-Fokus-Feldemissionsröntgenröhre nachfolgend als MBFEX-Röhre (MBFEX = Multibeam Field Emission X-Ray) bezeichnet wird. Ein solcher Computertomograph ist beispielsweise aus der US 7 751 528 B2 bekannt, der speziell für Röntgenaufnahmen der weiblichen Brust vorgesehen ist.

Brustkrebs ist die bei Frauen am häufigsten auftretende Krebsart. Ca. 10% der Frauen erkranken im Laufe ihres Lebens an Brustkrebs. Zwischen 30% und 40% dieser Patienten sterben daran, wobei die Mammographie ein wirksames Instrument zur Früherkennung von Brustkrebs ist. Durch Früherkennung kann die Brustkrebs-Mortalität signifikant verringert werden. In der Brustkrebs-Diagnostik wird zum Beispiel die Digitale Brust-Tomosynthese (DBT), ein 3D-Bildgebungsverfahren eingesetzt. Die DBT verfügt über eine verbesserte Leistung verglichen mit der digitalen Vollfeldmammographie (FFDM), die derzeit als der Goldstandard betrachtet wird. Jüngste klinische Studien haben gezeigt, dass die DBT-Technologie eine bessere Nachweisbarkeit für Verdichtungen in der weiblichen Brust bietet. Jedoch weist sie eine geringere Sensitivität für Verkalkungen auf, die kritisch für die Krebsidentifikation ist.

Weiter weisen bisherige Mammographie-Verfahren eine hohe Fehlerrate auf, wie beispielsweise eine sehr hohe positive (70 ∼ 90%) und negative (∼ 30%) Fehler-Rate. Die Eigenschaft der zweidimensionalen Mammographie erschwert es, einen Krebs von darüber liegendem Brustgewebe zu unterscheiden. Die Interpretation der Ergebnisse kann abhängig vom Radiologen variabel ausfallen, insbesondere im Falle einer verdichteten Brust. Eine höhere Fehler-Rate der falsch-positiven und falsch-negativen Testergebnisse treten auf, weil dichtes überlappendes Gewebe sich störend auf die Identifizierung von Anomalien im Zusammenhang von Tumoren auswirkt. Für junge Frauen mit vererbbaren Mutationen, die Voruntersuchungen in frühen Jahren beginnen möchten, sind diese Einschränkungen verbunden mit der Mammographie besonders problematisch.

Die Detektierbarkeit von Mikroverkalkungen durch ein DBT-System kann von vielen Faktoren abhängen und nachteilhaft beeinflusst werden, zum Beispiel durch den Detektortyp, die Rekonstruktion und den Aufnahmeparameter. Bewegungsunschärfe aufgrund der Quellen-/Detektor-Bewegung und der Bewegung des Patienten während der Aufnahme ist ein dominierender Faktor für die mangelnde räumliche Auflösung des DBT-Systems, also die Erkennung von kleinen Mikroverkalkungen. Außerdem ist die Patientin einer hohen Strahlenbelastung ausgesetzt.

Die Designs aller auf dem Markt befindlichen DBT-Scanner sind ähnlich. Eine konventionelle Röntgenröhre montiert auf einem rotierenden Arm bewegt sich in einem Bogen oberhalb der komprimierten Brust mit einem isozentrischen Bewegungsverlauf, um die Folge von Projektionsbildern innerhalb eines begrenzten Winkelbereichs zu erzeugen. Eine mechanische Konstruktion, zugehörige Steuerungssoftware und genaue Winkelmesseinrichtung sind notwendig, um die präzise Rotation der Röntgenröhre zu steuern. In dieser Art von Scannern kann ein gesamter Tomosynthese-Scan etwa 7 Sekunden bis zu mehr als 1 Minute dauern. Die Zeitdauer hängt von der Anzahl der erfassten Ansichten ab.

Computertomographen mit synchron zueinander rotierenden Röntgenröhren weisen jedoch erhebliche Nachteile auf. Die mechanische Instabilität, die durch Beschleunigung und Abbremsung der Quelle hervorgerufen wird, begrenzt die Geschwindigkeit, mit der die Röntgenröhre von einer Position zur nächsten Position bewegt werden kann. Je schneller die Abtastgeschwindigkeit ist, desto größer ist die Bewegungsunschärfe. Dieser Effekt ist besonders schwerwiegend für eine dichte Brust. Für eine gleichförmige und geometrisch präzise Rotation ist eine komplexe Mechanik mit einem hohen Platzbedarf erforderlich. Die mechanisch erfolgende Rotation bedingt weiter relativ langsame Rotationsgeschwindigkeiten und damit eine längere Aufnahmezeit. Solche Vorrichtungen sind sowohl in der Herstellung als auch durch die Störanfälligkeit der Mechanik im Unterhalt sehr kostenintensiv. Hervorzuheben ist insbesondere der hohe Energieverbrauch.

Eine Möglichkeit zur Behebung der genannten Nachteile, insbesondere die Scanzeit zu reduzieren und auch die räumliche Auflösung zu erhöhen, ist die Nutzung einer festen Reihenanordnung von Röntgenemittern. Bei einem solchen Computertomographen sind die Röntgenemitter auf den zu untersuchenden Körper ausgerichtet und werden jeweils einzeln elektrisch angesteuert. Die sequentielle Ansteuerung der Röntgenemitter ersetzt somit die bisher erforderliche Rotation einer Röntgenröhre. Dadurch können die Projektionsansichten ohne mechanische Bewegung erzeugt werden. Die durch die Quellenbewegung verursachte Bewegungsunschärfe kann so vollständig beseitigt werden. Eine Reihenanordnung von einzeln ansteuerbaren Röntgenemittern allgemein sind beispielsweise für einen Computertomographen in der US 7 233 644 B1 und der US 7 177 391 B2 mit mehreren Röntgenröhren, in der DE 10 2011 076 912 B4 mit einer Multi-Fokus-Röntgenröhre und in der DE 10 2009 017 649 A1 mit einer MBFEX-Röhre beschrieben.

Für Computertomographen mit einer festen Anordnung von Röntgenemittern sind solche Röntgenemitter verwendbar, welche als Feldemissions-Röntgenröhren ausgebildet sind. Solche Röntgenemitter weisen beispielsweise Kathoden auf, welche Kohlenstoffnanoröhren enthalten, wie dies ebenfalls in der US 7 751 528 B2 und in der DE 10 2009 017 649 A1 offenbart ist. Die Kohlenstoffnanoröhren dienen als Kaltkathoden, um Elektronen zu erzeugen, die dann beschleunigt werden, um auf der Anode Röntgenquellen zu erzeugen. Solche Röntgenemitter lassen sich besonders klein gestalten und in einer einzigen Vakuumröhre anordnen; eine solche Vorrichtung stellt eine MBFEX-Röhre dar, bei welcher wiederum eine kompaktere Bauform erzielbar ist.

Sowohl in der DE 10 2009 017 649 A1 als auch in der DE 10 2011 076 912 B4 ist eine MBFEX-Röhre offenbart, in welcher eine Mehrzahl von Kathoden auf eine gemeinsame Anode ausgerichtet ist.

In der DE 10 2009 017 649 A1 und in der US 2012 0286692 A1 wird eine Regelung die Kathoden der MBFEX-Röhre vorgeschlagen, bei welcher ein elektrischer Strom zwischen den Kathoden und einem Gitter bezüglich einer festgelegten Spannung eingestellt wird.

Die DE 10 2010 062 541 A1 beschreibt eine Mammografieanlage, die für die Durchführung einer Tomosynthese-Messung ausgestaltet ist. In diesem Fall ist eine Glühkathode zur thermischen Elektronenemission vorgesehen. Zusätzlich weist die bekannte Mammografieanlage zwei Multifokusröhren als Röntgenquelle auf. Kathoden der Röntgenemitter können als Dispenser-Kathoden, welche auch als Vorratskathoden bezeichnet werden, oder als Feldemitter ausgebildet sein.

Der Erfindung liegt die Aufgabe zugrunde, einen gegenüber dem Stand der Technik weiterentwickelten Computertomographen für die mammographische Röntgenbildgebung zur Verfügung zu stellen, mit welchem eine geringe Brennfleckgröße und eine verbesserte Untersuchungsmöglichkeit eines bestimmten Bereiches eines röntgentechnisch zu untersuchenden Querschnittes (ROI = Region of Interest) realisierbar ist.

Diese Aufgabe wird erfindungsgemäß durch einen Computertomographen mit den Merkmalen des Anspruchs 1 gelöst.

Der vorgeschlagenen Computertomograph für die mammographische Röntgenbildgebung weist eine MBFEX-Röhre und einen Flachbett-Röntgendetektor auf, wobei in der MBFEX-Röhre eine Mehrzahl von Kathoden reihenförmig und mindestens eine Anode fest angeordnet sind. Die Kathoden sind für die auf die jeweilige Anode gerichtete Feldemission von in einem elektrischen Feld extrahierten und beschleunigten Elektronen und die jeweiligen Anoden für eine gerichtete Emission von Röntgenstrahlen als Röntgenstrahlenbündel vorgesehen. Die Anzahl der Kathoden entspricht der Anzahl der Röntgenquellen, wobei auf der jeweiligen Anode mindestens eine Röntgenquelle erzeugbar ist. Hierbei sind die erzeugten Röntgenquellen ebenfalls reihenförmig in der MBFX-Röhre angeordnet und in ihrer Röntgen-Hauptemissionsrichtung auf die Detektor-Oberfläche des Flachbett-Röntgendetektors ausgerichtet. Der Flachbett-Röntgendetektor ist für eine Detektion von Röntgenstrahlen als Röntgenstrahlenbündel auf der Detektor-Oberfläche vorgesehen. An der MBFEX-Röhre ist beispielsweise an einem Röntgenfenster ein verstellbarer Kollimator angeordnet, mit welchem die Geometrie eines Röntgenstrahlenbündels einstellbar ist. Weiter ist die MBFEX-Röhre in Parallelrichtung zu dem Flachbett-Röntgendetektor verschiebbar. Der Flachbett-Röntgendetektor selbst weist eine verschiebbare als auch öffnungsverstellbare Röntgenblende auf. Mit der Röntgenblende ist ein Abbildungsbereich auf der Detektor-Oberfläche des Flachbett-Röntgendetektor auswählbar und verschiebbar.

Die Röntgenstrahlenbündel weisen jeweils eine Richtung mit der maximalen Intensität der emittierten Röntgenstrahlung auf, welche der jeweiligen Röntgen-Hauptemissionsrichtung entspricht. Eine solche Röntgen-Hauptemissionsrichtung ist bei allen Röntgenquellen gegeben, welche von einer Kugelstrahlquelle verschieden sind.

In dem vorgeschlagenen Computertomographen ist eine Röntgenquelle konstruktiv als eine begrenzte Fläche, beispielsweise als Ellipse oder als linearer Streifen, auf einer Anode realisierbar; dies ist wahlweise sowohl durch die Beschaffenheit der jeweiligen Kathode als auch durch Anpassung des elektrischen Feldes bewerkstelligbar. Durch eine geeignete Fokussierung der emittierten Elektronen als Elektronenstrahlenbündel ist die Form der Röntgenquelle wählbar, wobei die Fokussierung einer Anpassung des elektrischen Feldes in einem Volumenbereich zwischen der jeweiligen Kathode und der Anode entspricht. Mit dem vorgeschlagenen Computertomographen sind somit die Geometrie und die Strahlungsdichte eines Röntgenstrahlenbündels einstellbar. Ebenfalls ist der Wellenlängenbereich der emittierten Röntgenstrahlung und damit des Röntgenstrahlenbündels zusätzlich durch Fokussierung der Elektronenstrahlenbündel und Ausgestaltung der Kathoden einstellbar.

Bei dem vorgeschlagenen Computertomographen wird zur Röntgenbildaufnahme jeweils eine Kathode sequentiell elektrisch durch Ein- und Ausschalten angesteuert, wobei an alle Anoden während der Röntgenbildaufnahme eine gemeinsame gepulste oder zeitlich konstant gehaltene Gleichspannung anliegt. Durch die sequentielle Ansteuerung der einzelnen Kathoden wird im Gegensatz zu konventionellen Computertomographen nach dem Stand der Technik eine mechanische Rotation von Röntgenquellen ersetzt. Dabei ist das Untersuchungsobjekt zwischen der MBFEX-Röhre und dem Röntgen-Flachbettdetektor positioniert. Beispielsweise ist eine Brust einer Patientin auf einer Platte über der Röntgenblende aufgelegt, wobei die Platte durchlässig für Röntgenstrahlung ist.

In typischen Ausgestaltungen des Computertomographen ist die MBFEX-Röhre oberhalb des Detektors angeordnet. Jedoch sind auch andere Anordnungen mindestens einer Röntgenröhre und mindestens eines zugehörigen Detektors möglich, beispielsweise eine Anordnung einer Röntgenröhre unterhalb des zugehörigen Detektors, wie prinzipiell beispielsweise aus der DE 10 2010 011 663 A1 bekannt.

Mit der Röntgenblende ist ein Abbildungsbereich auf der Detektor-Oberfläche einstellbar, beispielsweise durch Verschiebung und Öffnung der Röntgenblende unterhalb einer auf einer Platte aufliegenden Brust. Bei einer Röntgenbildaufnahme ist das Untersuchungsobjekt unbewegt. Die MBFEX-Röhre ist in Parallelrichtung zu dem Flachbett-Röntgendetektor verschiebbar. Somit ist für jeden Verschiebungsschritt der MBFEX-Röhre in dem gewählten Abbildungsbereich eine Sequenz von Schaltungen der Kathoden und damit eine Röntgenbildaufnahme bewerkstelligbar, wobei in jedem Verschiebungsschritt die MBFEX-Röhre und das Untersuchungsobjekt eine Relativbewegung zueinander ausführen.

Beispielsweise kann eine Röntgenbildaufnahme dadurch erfolgen, dass nacheinander benachbarte Kathoden sequentiell elektrisch angesteuert werden. Ebenso können die Kathoden in beliebiger anderer Reihenfolge betrieben werden, wobei die Reihenfolge auch innerhalb der einzelnen optionalen Verschiebungsschritte in der Parallelrichtung variierbar ist.

Die Auswahl einer ROI kann sowohl durch Einstellung des Abbildungsbereiches mit der Röntgenblende, durch Festlegung der Verschiebungsschritte in einem Unterbereich auf dem Abbildungsbereich als auch dadurch erfolgen, dass nur diejenigen Kathoden elektrisch angesteuert werden, welche auf die ROI ausgerichtet sind. Aus den so erhaltenen Röntgenbildern, welche Projektionsaufnahmen darstellen, lassen sich mittels computergestützter Verfahren, wie Tomosynthese oder HEPC-Tomosynthese (HEPC = High-Energy Phase Contrast) oder gefilterter Rückprojektion (FBP = Filtered Back-Projection), Querschnittsansichten und Volumenstrukturen des untersuchten Objektes erzeugen.

Bei einer Projektion wird somit nur derjenige Einzelaufnahmebereich ausgewählt, welcher die für die computergestützte Bilderzeugung wesentlichen Informationen, das heißt Daten, enthält. Artefakte oder schlecht aufgelöste Bereiche werden damit vermieden. Insbesondere wird die für die computergestützte Bilderzeugung benötigte Zeit dadurch wesentlich verkürzt, dass auf der Detektor-Oberfläche des Flachbettdetektors der Detektionsbereich und damit der Abbildungsbereich auf die ROI mittels der Röntgenblende beschränkt wird.

Somit sind mit dem vorgeschlagenen Computertomographen bei gleichzeitig minimalem konstruktivem Aufwand hochaufgelöste Röntgenbildaufnahmen in einer bezüglich zum Stand der Technik verkürzten Aufnahmezeit möglich. Je mehr in der MBFEX-Röhre eine Mehrzahl von Kathoden und damit Röntgenquellen fest angeordnet sind, desto höher ist die in der gesamten ROI erreichbare Bildauflösung. Allgemein ist die Anzahl aller Kathoden in dem vorgeschlagenen Computertomographen mindestens gleich der Anzahl von Projektionen für eine solche computergestützte Bilderzeugung.

Bei einer vollumfänglichen Röntgenaufnahme eines Untersuchungsobjektes mit dem vorgeschlagenen Computertomographen, beispielsweise der weiblichen Brust, ist die MBFEX-Röhre vorzugsweise ortsfest gehalten. Bei einer Untersuchung einer ROI mit dem vorgeschlagenen Computertomographen, beispielsweise innerhalb einer weiblichen Brust wird dagegen die MBFEX-Röhre vorzugsweise über den gesamten Bereich der ROI schrittweise in der Parallelrichtung verschoben, wobei in jedem Verschiebungsschritt eine Röntgenbildaufnahme erfolgt.

Nachfolgend wird auf einzelne vorteilhafte Weiterbildungen des vorgeschlagenen Computertomographen bezüglich der Kathoden und Fokussierung der Elektronenstrahlenbündel in der MBFEX-Röhre eingegangen.

In einer möglichen Ausführung des vorgeschlagenen Computertomographen enthalten die Kathoden Kohlenstoffnanoröhren. Kohlenstoffnanoröhren weisen einen niedrigen Feldstärke-Schwellenwert von weniger als 2 V µm⁻¹ für die Feldemission von Elektronen auf. Aufgrund der somit verhältnismäßig geringen Leistungsaufnahme der Kathoden, welche für die Feldemission von Elektronen erforderlich ist, ist der Betrieb eines solchen Computertomographen mit einer Stromversorgung möglich, welche nur eine verhältnismäßig geringe Leistungsstärke aufweist.

Alternativ oder zusätzlich zu Kohlenstoffnanoröhren kann der Computertomograph sonstige Nanostäbchen, welche zur Emission von Elektronen ausgebildet sind, aufweisen. Beispielsweise handelt es sich hierbei um hohle oder massive Nanostäbchen, welche Metalloxide, Metallsulfide, Nitride, Carbide und/oder Silizium enthalten. Zum technischen Hintergrund wird beispielhaft auf die Veröffentlichung von Greta R. Patzke et al.: Oxidic Nanotubes and Nanorods - Anisotropic Modules for a Future Nanotechnology, Angew. Chem. Int. Ed. 2002, 41, 5000 - 5015 hingewiesen.

Sofern der erfindungsgemäße Elektronenemitter ein Sulfid enthält, kann es sich beispielsweise um ein Metallsulfid, insbesondere Molybdändisulfid, handeln. Als Nitride, aus welchen Nanostäbchen des Elektronenemitters vollständig oder teilweise aufgebaut sein können, sind insbesondere Bornitrid, Aluminiumnitrid, Kohlenstoffnitrid und Galliumnitrid zu nennen. Als Carbid ist insbesondere Siliziumcarbid zur Herstellung der Nanostäbchen, insbesondere Nanoröhren, geeignet. Ebenso sind Nanostäbchen, insbesondere in der Form von Nanoröhren, aus Silizium, optional mit Dotierungselementen, herstellbar. Auch die Verwendung von Nanostäbchen, welche Cer oder Lanthan enthalten, ist im Rahmen der Herstellung des Elektronenemitters des Computertomographen möglich. In diesem Zusammenhang wird beispielhaft auf das Dokument WO 2014/076693 A1 hingewiesen.

Ebenso können Kathoden, welche innerhalb des Computertomographen als Elektronenemitter fungieren, als Dispenserkathoden ausgebildet sein. Derartige Kathoden sind grundsätzlich zum Beispiel aus der DE 10 2011 076 912 B4 bekannt.

In einer weiteren Ausführung des vorgeschlagenen Computertomographen weist die MBFEX-Röhre eine fest angeordnete Gittervorrichtung auf. In der Gittervorrichtung ist eine allen Kathoden gemeinsame Extraktionsgitterelektrode oder jeder einzelnen Kathode jeweils eine separate Extraktionsgitterelektrode zugeordnet. Hierbei ist jede Extraktionsgitterelektrode direkt über den Kathoden angeordnet und für die Feldextraktion von Elektronen aus den Kathoden vorgesehen. Die Extraktionsgitterelektroden sind vorzugsweise bei Nichtbetrieb geerdet oder vorzugsweise galvanisch mit den Kathoden schaltbar. Weiter sind die Extraktionsgitterelektroden von allen anderen Komponenten der MBFEX-Röhre galvanisch getrennt, wodurch besonders vorteilhaft ein Durchschlagen des elektrischen Feldes auf diese Komponenten unterbunden und das Strahltransportverhalten von der elektrischen Spannung zwischen der Kathode und der Extraktionsgitterelektrode weitestgehend entkoppelt ist. Die Extraktionsgitterelektroden sind unabhängig von den Kathoden oder wahlweise sequentiell zusammen mit den Kathoden schaltbar.

Bei einer Röntgenbildaufnahme sind die Extraktionsgitterelektroden als Elektroden mit einem positiven elektrischen Potential, relativ zur Kathode, geschaltet; hierdurch wird die Feldemission von Elektronen wesentlich verbessert und auch einem elektrischen Überschlag in der näheren Umgebung der Kathoden entgegengewirkt. Bei der Hochspannungskonditionierung der MBFEX-Röhre sind die Extraktionsgitterelektroden ebenfalls als Elektroden mit einem positiven elektrischen Potential geschaltet, wobei die Kathoden ausgeschaltet sind. Hierdurch sind die Kathoden gegenüber einer Ionenbombardierung geschützt.

In einer Weiterbildung der letzten Ausführungsform des vorgeschlagenen Computertomographen weist die Gittervorrichtung direkt über jeder Extraktionsgitterelektrode für jede einzelne Kathode jeweils eine separat zugeordnete Fokussierungselektrode auf. Die Fokussierungselektroden sind vorzugsweise bei Nichtbetrieb geerdet. Weiter sind die Fokussierungselektroden von allen anderen Komponenten der MBFEX-Röhre galvanisch getrennt.

Die Fokussierungselektroden sind für die Fokussierung der extrahierten Elektronen als Elektronenstrahlenbündel vorgesehen. Die Fokussierungselektroden sind sequentiell zusammen mit den zugeordneten Kathoden wahlweise elektronisch ansteuerbar.

Bei einer Röntgenbildaufnahme sind die Fokussierungselektroden jeweils als Elektroden mit einem positiven oder negativen elektrischen Potential geschaltet, je nachdem ob eine Fokussierung oder eine Defokussierung erreicht werden soll. Sowohl durch das Vorzeichen und den Betrag des elektrischen Potentials als auch durch die geometrische Form der jeweiligen Fokussierungselektrode ist eine Fokussierung des Elektronenstrahlenbündels einstellbar. Bei einer festgelegten geometrischen Form der Fokussierungselektroden sind mit dem vorgeschlagenen Computertomographen mit jeder Einstellung eines elektrischen Potentials jeweils Röntgenbildaufnahmen mit unterschiedlichen Röntgenstrahlenbündeln bewerkstelligbar. Hierbei weisen alle Fokussierungselektroden ein jeweils gleiches elektrisches Potential auf, so dass alle bei einer Röntgenbildaufnahme sequentiell erzeugten Röntgenstrahlenbündel dieselbe Geometrie, Strahlungsdichte und denselben Wellenlängenbereich aufweisen. Insbesondere ist so mit den Fokussierungselektroden die Brennfleckgröße der Röntgenstrahlenbündel einstellbar.

Bei der Hochspannungskonditionierung der MBFEX-Röhre sind die Kathoden ausgeschaltet und die Extraktionsgitterelektroden mit einem positiven Potential beaufschlagt. Durch diese zusätzliche Schutzschaltungsweise sind die Kathoden gegenüber einer lonenbombardierung noch vorteilhafter abschirmbar.

In einer anderen Ausführungsform weist der vorgeschlagene Computertomograph mehr als eine reihenförmige Anordnung von gleichen Kathoden oder von Kathoden verschiedener Sorten auf. In dieser Ausführungsform umfasst eine reihenförmige Anordnung nicht mehr als eine Sorte von Kathoden. Auf jeweils eine Anode sind Kathoden von jeder Sorte ausgerichtet. Bei einer Röntgenbildaufnahme ist beispielsweise jeweils nur eine Sorte von Kathoden sequentiell ansteuerbar. In abgewandelten Ausführungsformen können innerhalb ein und derselben Reihe von Kathoden verschiedene Sorten von Kathoden vorhanden sein. Der Begriff "Sorte von Kathoden" kann sich im einfachsten Fall lediglich auf die Fläche beziehen, welche eine Kathode auf einem typischerweise keramischen Träger einnimmt. In einem solchen Fall können sämtliche Kathoden der diversen Sorten ansonsten gleichartig gestaltet sein. In anderen Fällen unterscheiden sich die verschiedenen Kathoden beispielsweise hinsichtlich ihres Materials oder sonstiger Merkmale, wogegen die von außen erkennbaren Konturen der diversen Sorten von Kathoden in solchen Fällen einheitlich sein können.

Beispielsweise sind die Kathoden quadratisch, rechteckig, rund oder elliptisch ausgebildet. Sofern sich die Kathoden bezüglich ihrer Flächengeometrie voneinander unterscheiden, können Unterschiede hinsichtlich der Flächengeometrie und/oder Flächengröße gegeben sein. Liegen beispielsweise rechteckige Kathoden vor, so können sich die Kathoden durch die Rechteckflächengröße voneinander unterscheiden. Ebenso sind unterschiedliche Längen-/Breitenverhältnisse verschiedener Kathoden möglich.

Kathoden mit unterschiedlicher Flächengeometrie oder Flächengröße erzeugen unterschiedliche Elektronenstrahlenbündel. Somit sind allein durch die jeweilige Wahl der Kathoden auf der betreffenden Anode unterschiedliche Röntgenquellen und damit unterschiedliche Röntgenstrahlenbündel unterschiedlicher Geometrien erzeugbar, wenn beispielsweise die Fokussierungselektroden in ihrer Konstruktionsform und Anordnungen über den Kathoden und Extraktionsgitterelektroden identisch sind und ein gleiches elektrisches Potential aufweisen. Mit dem Kollimator ist dann eine Verfeinerung der Geometrie der jeweils gewählten Röntgenstrahlenbündel realisierbar.

Beispielsweise sind bei ansonsten gleichen Extraktionsgitterelektroden und Fokussierungselektroden für eine computergestützte Röntgenbildgebung mittels HPEC-Tomosynthese mit dem vorgeschlagenen Computertomographen bevorzugt Kathoden mit einer kleineren Fläche als für eine computergestützte Röntgenbildgebung mittels Tomosynthese ausgewählt. Hierdurch ist bei der Röntgenbildgebung mittels HPEC-Tomosynthese besonders vorteilhaft die Brennfleckgröße herabgesetzt und damit eine bessere Bildauflösung der ROI erzielbar.

Sind in dieser Ausführungsform des vorgeschlagenen Computertomographen verschiedene Kathoden aus unterschiedlichen Materialien gefertigt, so unterscheiden sich diese auch bezüglich der Energie der emittierten Elektronen. Dies gilt auch dann, wenn den Kathoden hinsichtlich ihrer Konstruktionsweise und Anordnungsweise gleiche Extraktionsgitterelektroden zugeordnet sind und an allen Extraktionsgitterelektroden bei einer Röntgenbildaufnahme ein gleiches, relativ zur Kathode positives elektrisches Potential angelegt ist. Beispielsweise weisen Kathoden einer ersten Sorte Kohlenstoffnanoröhren und Kathoden einer anderen Sorte Spitzen aus Wolfram oder Molybdän auf.

Alternativ oder zusätzlich ist durch Ansteuerung der Kathoden mit unterschiedlichen elektrischen Spannungen oder unterschiedlichen elektrischen Strömen die Energie der emittierten Elektronen und damit die Energie der emittierten Röntgenstrahlung einstellbar. Beispielsweise unterscheiden sich die Formen der Kathoden darin, dass diese jeweils für eine unterschiedliche Ansteuerung bezüglich des elektrischen Stroms oder der elektrischen Spannung vorgesehen sind. Beispielsweise weist die MBFEX-Röhre des vorgeschlagenen Computertomographen zwei Sorten von Kathoden auf, wobei die eine Sorte für den Betrieb mit einem stärkeren und die andere Sorte für den Betrieb mit einem schwächeren gepulsten Gleichstrom vorgesehen ist. Beispielsweise weist die MBFEX-Röhre des vorgeschlagenen Computertomographen zwei Sorten von Kathoden auf, wobei beide Sorten für den Betrieb mit einer gepulsten Rechteck-Gleichspannung oder einem gepulsten Rechteck-Gleichstrom, jedoch mit unterschiedlicher Schaltfrequenz, vorgesehen sind.

Durch eine teilweise oder vollumfassende Kombination von unterschiedlichen Flächengeometrien, Flächengrößen, unterschiedlicher Materialbeschaffenheit und bezüglich Strom oder Spannung unterschiedlicher Ansteuerungen sind in der MBFX-Röhre des vorgeschlagenen Computertomographen eine Vielzahl unterschiedlicher Formen von Kathoden realisierbar. Beispielsweise ist eine Sorte von Kathoden für die Realisierung von Röntgenbildaufnahmen über Tomosynthese und die andere Sorte für die Realisierung von Röntgenbildaufnahmen über HEPC-Tomosynthese vorgesehen.

In einer weiteren bevorzugten Ausführungsform ist der vorgeschlagenen Computertomograph dahingehend weitergebildet, dass jeder reihenförmigen Anordnung von Kathoden Extraktionsgitterelektroden und Fokussierungselektroden jeweils eines Typs zugeordnet sind, wobei sich die reihenförmigen Anordnungen von Kathoden durch mindestens einen Typ von Extraktionsgitterelektroden und / oder mindestens einen Typ von Fokussierungsgitterelektroden unterscheiden.

Somit sind selbst bei mehreren Anordnungen aus Kathoden einer gleichen Sorte durch Wahl unterschiedlicher Typen von Extraktionsgitterelektroden und / oder unterschiedlichen Typen von Fokussierungsgitterelektroden sowohl die Geometrie, die Strahlungsdichte als auch der Wellenlängenbereich eines Röntgenstrahlenbündels einstellbar, selbst wenn sich die Typen der Extraktionsgitterelektroden und / oder Fokussierungselektroden lediglich durch einen Betrieb mit einer unterschiedlichen elektrischen Spannung oder mit einem unterschiedlichen elektrischen Strom unterscheiden.

Beispielsweise sind unterschiedliche Typen von Extraktionsgitterelektroden durch unterschiedliche Konstruktionsformen und / oder durch unterschiedliche Anordnungen über den Kathoden gegeben. Beispielsweise sind unterschiedliche Typen von Fokussierungselektroden durch unterschiedliche Konstruktionsformen und / oder durch unterschiedliche Anordnungen über den Kathoden und Extraktionsgitterelektroden realisierbar.

In einer bevorzugten Ausführung dieser Weiterbildung des vorgeschlagenen Computertomographen weist die MBFX-Röhre mehrere Anordnungen aus Kathoden einer gleichen Sorte mit Extraktionsgitterelektroden gleichen Typs auf, wobei sich die reihenförmigen Anordnungen von Kathoden hinsichtlich der Typen von Fokussierungsgitterelektroden unterscheiden. In dieser Ausführungsform des vorgeschlagenen Computertomographen sind sowohl die Geometrie, die Strahlungsdichte als auch der Wellenlängenbereich eines Röntgenstrahlenbündels allein durch die Wahl und den Betrieb der Fokussierungselektroden bestimmbar.

Die vielfältigen Variationsmöglichkeiten, welche insbesondere die Kathoden und deren Ansteuerung betreffen, ermöglichen den Betrieb des Computertomographen in einem Multi-Dosis-Modus. In einem solchen Modus wird beispielsweise zu einem bestimmten Zeitpunkt eine Kathode derart angesteuert, dass sie einen Elektronenstrom von 10 mA emittiert, wobei die Anodenspannung auf 20 kV eingestellt ist. Diese Werte bleiben beispielsweise über eine Pulsdauer von 10 ms konstant. Bereits der nächste Puls, welcher zum Beispiel 100 ms dauert, kann mit einem mehrfach höheren Elektronenstrom von 30 mA emittiert werden, wobei zum Beispiel eine Anodenspannung in Höhe von 100 kV eingestellt ist. Die damit gegebene Flexibilität in der Ansteuerung des Computertomographen ermöglicht eine entsprechend flexible, auf den Einzelfall abstimmbare, insgesamt dosisarme röntgentechnische Bildgebung mit zugleich hoher Abbildungsqualität.

Mit dem vorgeschlagenen Computertomographen in den zuvor dargelegten Weiterbildungen sind somit bei einer Röntgenbildaufnahme jeweils Röntgenstrahlenbündel mit einer identischen Brennfleckgröße realisierbar, wobei die jeweilige Brennfleckgröße für jede Röntgenbildaufnahme durch vorherige Einstellung der Fokussierung und Wahl einer Anordnung von Kathoden einstellbar ist. Die jeweilige Brennfleckgröße ist hierbei auch durch die jeweilige Sorte der Kathoden, durch den jeweiligen Typ der Gitterextraktionselektrode und vor allem durch den jeweiligen Typ der Fokussierungselektrode bestimmt.

Für eine computergestützte Röntgenbildgebung sowohl mittels Tomosynthese als auch mittels HPEC-Tomosynthse liegt auf der betreffenden Anode vorzugsweise einem ersten Teilbetriebsmodus entsprechend ein zeitlich konstantes positives elektrisches Potential an, wobei die Kathoden vorzugsweise mit einem gleichförmig gepulsten negativen elektrischen Rechteck-Potential und die Extraktionsgitterelektroden vorzugsweise mit einem gleichförmig gepulsten positiven elektrischen Rechteck-Potential, bezogen auf das Potential der Kathode, beaufschlagt sind. Somit fließt bei diesen bevorzugten Betriebsmodi ein gleichförmig gepulster elektrischer Gleichstrom.

Für eine computergestützte Röntgenbildgebung mittels Tomosynthese ist bevorzugt eine niedrigere Spannung zwischen der betreffenden Anode und den Kathoden, aber ein stärkerer Strom als für eine computergestützte Röntgenbildgebung mittels HPEC-Tomosynthese gewählt. Durch diese drei bevorzugten Betriebsmodi sind sowohl die betreffenden Anoden als auch die Kathoden vor Überhitzung hinreichend geschützt.

Für eine computergestützte Röntgenbildgebung mittels Tomosynthese sind bevorzugt Elektronenstrahlenbündel und damit Röntgenstrahlenbündel geringerer Energie als für eine computergestützte Röntgenbildgebung mittels HPEC-Tomosynthese gewählt, so dass für eine Tomosynthese die Fokussierungselektroden bevorzugt ein niedrigeres elektrisches Potential als für eine computergestützte Röntgenbildgebung mittels HPEC-Tomosynthese aufweisen. Die Fokussierungselektroden sind vorzugsweise sowohl bei Röntgenaufnahmen als auch bei einer Konditionierung der MBFEX-Röhre mit einem zeitlich konstanten elektrischen Potential beaufschlagt.

Nachfolgend wird auf einzelne vorteilhafte Weiterbildungen des vorgeschlagenen Computertomographen bezüglich der Anode der MBFEX-Röhre eingegangen.

In einer weiteren bevorzugten Ausführung des vorgeschlagenen Computertomographen sind die Kathoden in der MBFEX-Röhre bezüglich ihrer Elektronen-Hauptemissionsrichtung auf eine gemeinsame feste Anode ausgerichtet. Vorzugsweise sind hierbei die Kathoden in der Weise auf die Anode ausgerichtet, so dass auf der Anode eine reihenförmige Anordnung von Röntgenstrahlungsquellen gegeben ist; diese Röntgenstrahlungsquellen entsprechen damit Röntgenemittern. Somit ist bei dieser Ausführung des vorgeschlagenen Computertomographen der schaltungstechnische Aufwand erheblich reduziert. Weiter ist so eine besonders kompakte und gewichtsreduzierte Bauform der MBFEX-Röhre bewerkstelligbar.

Besonders vorteilhaft ist in einer Ausführung des vorgeschlagenen Computertomograph die gemeinsame Anode, zu welcher die Kathoden zugeordnet sind, bogenförmig ausgebildet und konkav auf die Detektoroberfläche ausgerichtet. Somit sind mit einer solchen Anode die Röntgenquellen ebenfalls bogenförmig ausgebildet und konkav auf die Detektoroberfläche ausgerichtet. Mit einer solchen Anode sind tote Winkel um die Parallelrichtung vermeidbar. Dies gestattet bei einem Untersuchungsobjekt eine hoch aufgelöste Abbildung einer ROI selbst für solche Bereiche, welche teilweise von einer stark Röntgenstrahlung absorbierenden Teilschicht verdeckt sind.

Die Anode ist unabhängig davon, ob sie insgesamt eine langgestreckte, gerade, insbesondere zylindrische Form oder eine gebogene Form aufweist, vorzugsweise als flüssigkeitsgekühlte Anode ausgebildet. Hierbei strömt ein Kühlmittel, insbesondere in Form eines elektrisch nicht leitfähigen Öles, beispielsweise Siliconöles, durch einen in Längsrichtung der Anode verlaufenden Kanal, wobei der Rückfluss des Kühlmittels aus der Anode heraus durch einen weiteren, zum ersten Kanal konzentrischen Kanal erfolgt, so dass sich lediglich an einem Ende der Anode ein Kühlmittelanschluss für die Ein- und Ausleitung des Kühlmittels befindet. Diese Bauform hat den Vorteil, dass der genannte Anschluss für Kühlmittel mittels einer einzigen Leitungsanordnung mit einer Hochspannungsdurchführung der Röntgenröhre verbunden sein kann, womit die Anzahl der Hochspannungsdurchführungen minimiert ist.

Nachfolgend wird auf einzelne vorteilhafte Weiterbildungen des vorgeschlagenen Computertomographen bezüglich der bewerkstelligbaren Einstellungen der MBFEX-Röhre, des Röntgenstrahlenbündels und des Abbildungsbereiches eingegangen.

In einer solchen Weiterbildung des vorgeschlagenen Computertomographen ist mit dem Kollimator ein Röntgenstrahlenbündel als ein Röntgen-Kegelbündel mit einem kreisförmigen oder ellipsenförmigen Röntgen-Auftreffbereich oder als ein Röntgen-Fächerbündel mit einem linienförmigen Auftreffbereich einstellbar. Der Röntgen-Auftreffbereich des Röntgenstrahlenbündels bezieht sich hierbei auf die Detektor-Oberfläche, auf welche das Röntgenstrahlenbündel auftrifft. Der Röntgen-Auftreffbereich des Röntgenstrahlenbündels deckt die Detektor-Oberfläche vollständig oder zumindest teilweise ab. Bei einem Röntgenstrahlbündel in Form eines Röntgen-Kegelbündels entspricht die Röntgen-Hauptemissionsrichtung der Kegelachse. Bei einem Röntgenstrahlbündel in Form eines Röntgen-Fächerbündels verläuft die Röntgen-Hauptemissionsrichtung durch eine den Röntgen-Auftreffbereich längsseitig halbierende Fächerebene. Zur Röntgenbildaufnahme des gesamten Untersuchungsobjektes, beispielsweise einer weiblichen Brust, sind die Röntgenstrahlenbündel vorzugsweise in Form von Röntgen-Kegelbündeln eingestellt, wobei die MBFEX-Röhre ortsfest gehalten ist.

Weitergehend ist der Computertomograph so ausgestaltet, dass der Röntgen-Abbildungsbereich synchron mit der MBFEX-Röhre in der Parallelrichtung verschiebbar und das Röntgenstrahlenbündel als Röntgen-Fächerbündel einstellbar ist. Hierbei ist die Fächerebene senkrecht zu der Parallelrichtung. Der Auftreffbereich deckt den Abbildungsbereich vollständig ab und ist rechteckig. Die Fächerebene ist hierbei parallel zur Längsseite des Röntgen-Abbildungsbereiches und halbiert den Röntgen-Abbildungsbereich geometrisch. In dieser Ausführungsform ist die MBFEX-Röhre des vorgeschlagenen Computertomographen synchron mit der Röntgenblende ansteuerbar. Zur Röntgenbildaufnahme einer ROI eines Untersuchungsobjektes, beispielsweise einer weiblichen Brust, sind die Röntgenstrahlenbündel vorzugsweise in Form von Röntgen-Fächerbündeln eingestellt; hierbei ist für jeden Verschiebungsschritt der MBFEX-Röhre und der Röntgenblende in dem für die ROI gewählten Röntgen-Abbildungsbereich eine Sequenz von Schaltungen der Kathoden und damit eine Röntgenbildaufnahme bewerkstelligbar.

In einer anderen Ausführungsform ist die MBFEX-Röhre bezüglich der Detektoroberfläche in einer Senkrechtrichtung (typischerweise als y-Richtung bezeichnet) abstandsverstellbar. Somit ist im Sinne einer Justierung der Auftreffbereich des Röntgenstrahlenbündels bezüglich des Abbildungsbereiches vor Durchführung der computergestützten Röntgenbildaufnahme einstellbar. Im Vergleich zu herkömmlichen Lösungen sind damit auch im Wege einer HEPC-Tomosynthese durch die Verstellung der MBFEX-Röhre Röntgenaufnahmen mit höherer Anodenspannung und gleichzeitig geringerem Emissionsstrom realisierbar.

Im Gegensatz zu bekannten Computertomographen mit einer rotierenden Röntgenröhre ist bei Röntgenbildaufnahmen mit dem vorgeschlagenen Computertomographen eine Brennfleckvergrößerung um die Axialrichtung aufgrund bewegter Komponenten prinzipbedingt ausgeschlossen.

Der vorgeschlagene Computertomograph, insbesondere in seinen Weiterbildungen, zeichnet sich durch eine sehr kompakte und robuste Bauweise aus. Der vorgeschlagene Computertomograph, insbesondere mit einer MBFEX-Röhre, welche Kaltkathoden mit Kohlenstoffnanoröhren aufweist, weist im Vergleich zu den aktuell auf dem Markt erhältlichen Computertomographen folgende Vorteile auf:
- Reduktion der Strahlungsdosis für die Patienten,
- Steigerung der Sensitivität und Spezifität von bildgebenden Geräten,
- weniger Gewicht und Stellfläche,
- Verbesserung der Qualität bzw. Senkung der Kosten (speziell die Anschaffungs- und Betriebskosten für solche medizinischen Bildgebungssysteme) von Gesundheitsfürsorge-Dienstleister.

Die Verwendung des vorgeschlagenen Computertomographen ist keineswegs auf die medizinische Diagnostik beschränkt. Der vorgeschlagenen Computertomograph ist beispielsweise auch für die Röntgenbildgebung von nichtbelebten Objekten, beispielsweise zur Werkstückprüfung oder Produktprüfung oder zur Inhaltsprüfung von verschlossenen Behältern, geeignet. Insbesondere die MBFEX-Röhre des vorgeschlagenen Computertomographen in der Ausführung, bei welcher eine Mehrzahl von Kathoden einer gemeinsamen Anode zugeordnet sind, ist auch für andere Computertomographen verwendbar.

Nachfolgend wird der vorgeschlagene Computertomograph anhand einer Zeichnung näher erläutert, in welcher drei Ausführungsbeispiele zusammengefasst sind. Hierin zeigt:
- Fig. 1: ein erstes Ausführungsbeispiel eines Computertomographen **1** in schematischer Ansicht seiner MBFEX-Röhre **20,**
- Fig. 2: das erste Ausführungsbeispiel eines Computertomographen **1** mit Darstellung eines Röntgenstrahlenbündels **b** in Form eines Kegelbündels **c,**
- Fig. 3: das erste Ausführungsbeispiel eines Computertomographen **1** mit Darstellung eine Röntgenstrahlenbündels **b** in Form eines Fächerbündels **f,**
- Fig. 4: eine Teilansicht einer Gittervorrichtung **50** der MBFEX-Röhre **20** des ersten Ausführungsbeispiels eines Computertomographen **1,**
- Fig. 5: eine Teilansicht der Gittervorrichtung **50** der MBFEX-Röhre **20** eines zweiten Ausführungsbeispiels eines Computertomographen **1,**
- Fig. 6: ein drittes Ausführungsbeispiel eines Computertomographen **1** mit reihenförmig und alternierend versetzt angeordneten Kathoden **41, 42** zweier unterschiedlicher Sorten.

Alle nachfolgend anhand einer Zeichnung erläuterten Ausführungsbeispiele des vorgeschlagenen Computertomographen **1** sind für die mammographische Röntgenbildgebung vorgesehen. Alle nachfolgend erläuterten Ausführungsbeispiele des vorgeschlagenen Computertomographen **1** weisen eine MBFEX-Röhre **20** und einen Flachbett-Röntgendetektor **30** auf. Eine rechteckige Detektoroberfläche **D** des Flachbett-Röntgendetektors **30** ist für die Detektion von Röntgenstrahlen vorgesehen.

Die MBFEX-Röhre **20** weist in allen Ausführungsbeispielen eine Vakuumröhre **21,** ein Röntgenfenster **22** und einen Kollimator **23** auf, wobei mit dem Kollimator die Geometrie eines Röntgenstrahlenbündels **b** einstellbar ist. Die Röntgenstrahlenbündel **b** weisen eine Richtung mit der maximalen Intensität der emittierten Röntgenstrahlung auf, welche einer Röntgen-Hauptemissionsrichtung **h** entspricht. In beiden Ausführungsbeispielen des vorgeschlagenen Computertomographen **1** ist mit dem Kollimator **23** das Röntgenstrahlenbündel **b** wahlweise als ein Röntgen-Kegelbündel **c** mit einem kreisförmigen oder ellipsenförmigen Röntgen-Auftreffbereich **B** oder als ein Röntgen-Fächerbündel **f** mit einem linienförmigen Röntgen-Auftreffbereich **B** einstellbar ist, wobei das Röntgen-Fächerbündel **f** eine den Röntgen-Auftreffbereich **B** längsseitig halbierende Röntgen-Fächerebene **F** aufweist. Der Röntgen-Auftreffbereich **B** deckt die Detektoroberfläche **D** teilweise oder vollständig ab.

In allen drei Ausführungsbeispielen des vorgeschlagenen Computertomographen **1** sind die Kathoden **40, 41, 42** jeweils in reihenförmigen Anordnungen in Form eines Bogens fest angeordnet. In dem ersten Ausführungsbeispiel weist die MBFEX-Röhre **20** eine Anordnung mit einer Mehrzahl von gleichförmigen Kathoden **40** auf. In dem zweiten Ausführungsbeispiel weist die MBFEX-Röhre **20** zwei Anordnungen mit jeweils einer gleichen Mehrzahl von Kathoden **41, 42** zweier unterschiedlicher Sorten auf, wobei jede der beiden Anordnungen jeweils eine Sorte von Kathoden **41, 42** aufweist und die Kathoden **41** der ersten Sorte vor den Kathoden **42** der zweiten Sorte angeordnet sind. Das dritte Ausführungsbeispiel des vorgeschlagenen Computertomographen **1** unterscheidet sich von dem zweiten lediglich dadurch, dass die Kathoden **41, 42** zwar reihenförmig, aber alternierend versetzt angeordnet sind. In allen drei Ausführungsbeispielen weisen die Kathoden mehrwandige Kohlenstoffnanoröhren in einer überwiegend senkrechten Vorzugsrichtung zur jeweiligen Kathodenoberfläche auf und sind rechteckförmig ausgebildet. Die Kathoden **41** der ersten Sorte und die Kathoden **42** der zweiten Sorte des zweiten und dritten Ausführungsbeispiels unterscheiden sich in ihrer Flächengröße.

In allen Ausführungsbeispielen sind die Kathoden **40, 41, 42** für die Feldemission von Elektronen vorgesehen, auf eine gemeinsame Anode **6** ausgerichtet und wahlweise mit einem gleichförmig gepulsten negativen Potential von bis zu 4 KV beaufschlagbar.

In allen Ausführungsbeispielen ist die Anode **6** bogenförmig ausgebildet, konkav auf die Detektoroberfläche **D** ausgerichtet und in der Vakuumröhre **21** fest angeordnet. In allen Ausführungsbeispielen sind die Kathoden **40, 41, 42** in der Weise auf die Anode **6** ausgerichtet, so dass auf der Anode **6** eine reihenförmige Anordnung von Röntgenstrahlenquellen **Q** erzeugbar ist, wobei die Röntgenquellen **Q** ebenfalls bogenförmig ausgebildet und konkav auf die Detektoroberfläche **D** ausgerichtet sind. Bei einer Röntgenbildaufnahme ist in allen drei Ausführungen des vorgeschlagenen Computertomographen **1** ist eine Röntgenbildaufnahme durch eine sequentielle Ansteuerung der Kathoden **40, 41, 42** realisierbar.

In allen drei Ausführungsbeispielen weist die MBFEX-Röhre **20** eine Gittervorrichtung **50** auf, wobei die Gittervorrichtung **50** auf die Anode **6** ausgerichtet ist. Die Gittervorrichtung **50** ist zwischen den Kathoden **40, 41, 42** und der Anode **6** in der Vakuumröhre **21** angeordnet. Die Gittervorrichtung **50** aller drei Ausführungsbeispiele weist mindestens eine Extraktionsgitterelektrode **51, 53, 54** und mindestens eine Form von Fokussierungselektroden **52, 55, 56** auf.

Die Extraktionsgitterelektroden **51, 53, 54** sind direkt über den Kathoden **40, 41, 42** fest angeordnet und zur Feldextraktion von Elektronen aus den Kathoden **40, 41, 42** vorgesehen. Die Fokussierungselektroden **52, 55, 56** sind direkt über jeder Extraktionsgitterelektrode **51, 53, 54** ebenfalls fest angeordnet, der Anode **6** zugewandt und für die Fokussierung der extrahierten Elektronen als ein Elektronenstrahlbündel **a** auf die jeweilige zu erzeugende Röntgenstrahlungsquelle **Q** vorgesehen.

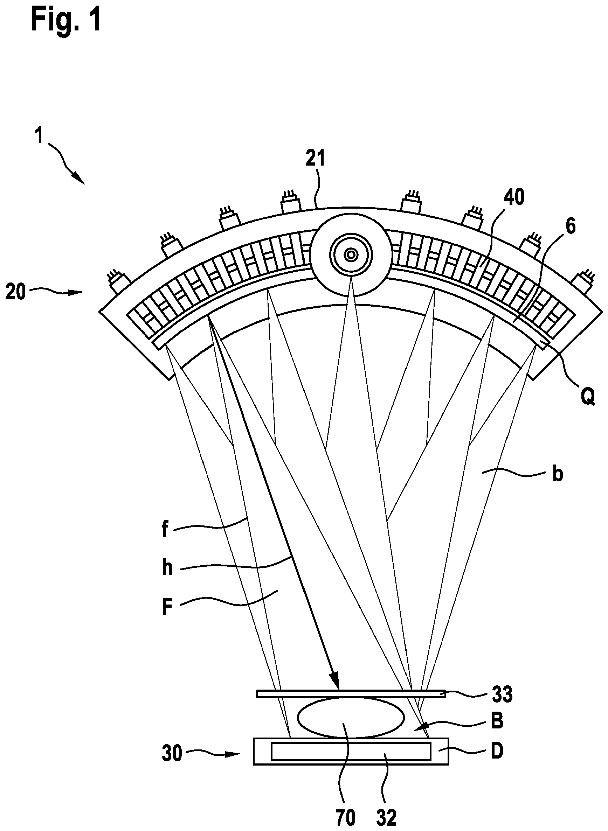

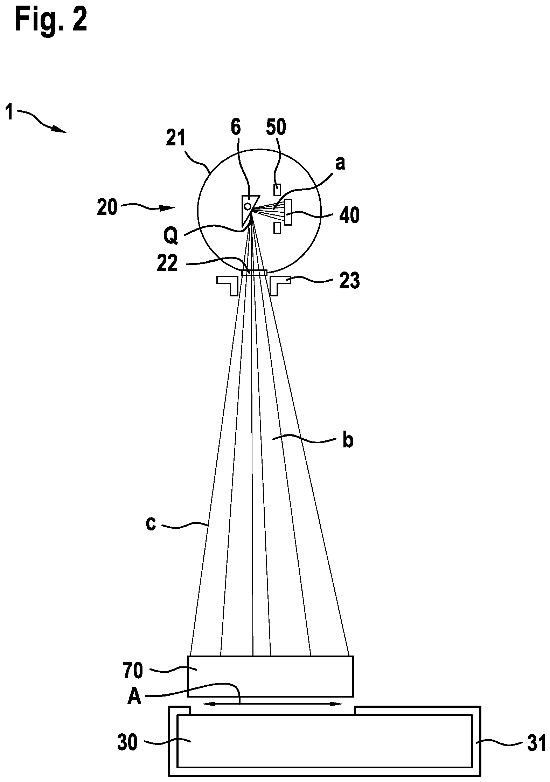

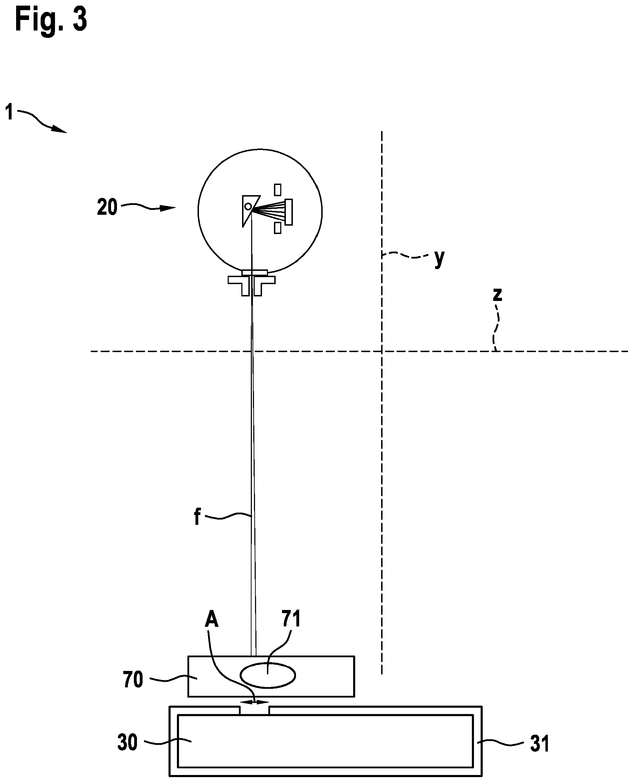

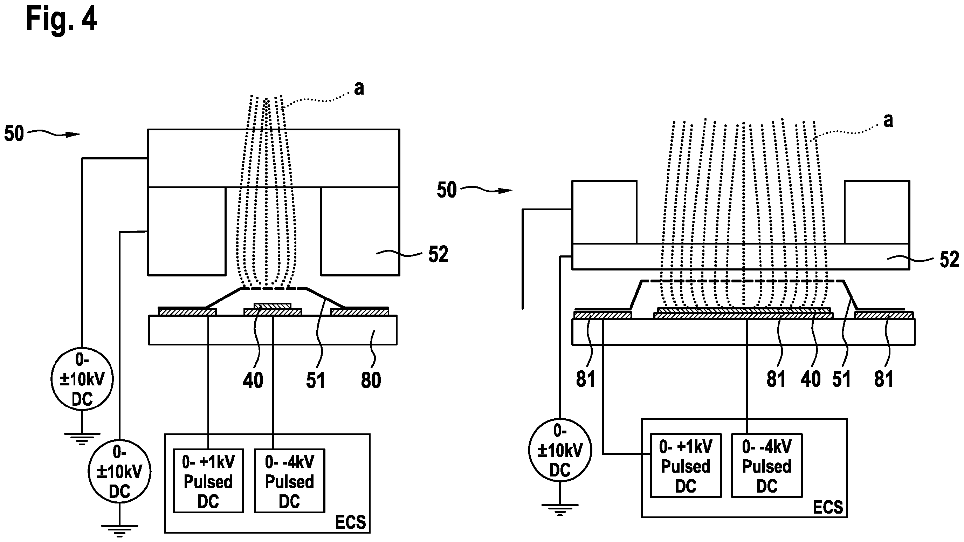

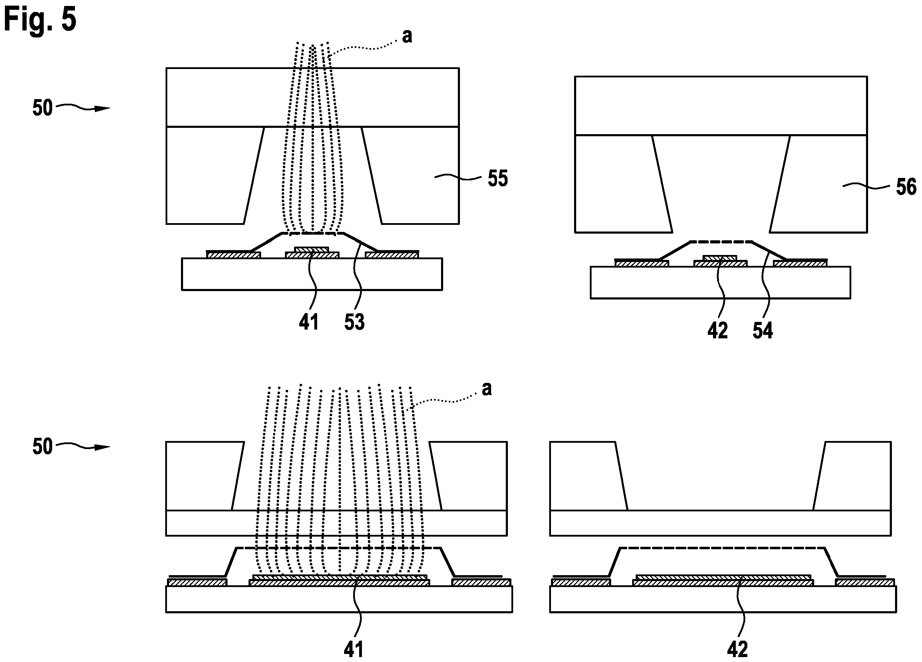

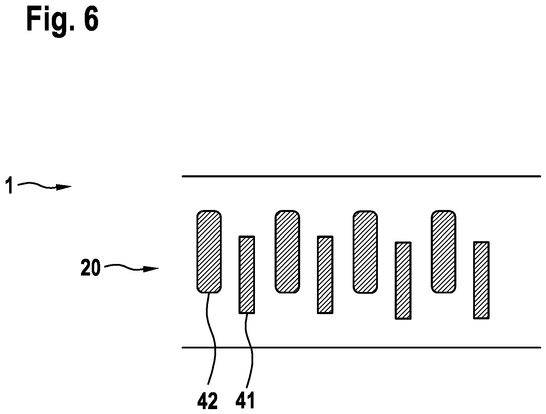

In dem ersten Ausführungsbeispiel weist die Gittervorrichtung **50** eine allen Kathoden **40** gemeinsame Extraktionsgitterelektrode **51** auf, wobei jeder einzelnen Kathode **40** separat eine einzelne Fokussierungselektrode **52** zugeordnet ist. In dem zweiten und dritten Ausführungsbeispiel weist die Gittervorrichtung **50** eine den Kathoden **41** der ersten Sorte gemeinsame Extraktionsgitterelektrode **53** einer ersten Form und eine den Kathoden **42** der zweiten Sorte gemeinsame Extraktionsgitterelektrode **54** einer zweiten Form auf, wobei jeweils jeden einzelnen Kathode **41** der ersten Sorte separat eine einzelne Fokussierungselektrode **55** einer ersten Form und jeweils jeden einzelnen Kathode **42** der zweiten Sorte separat eine einzelne Fokussierungselektrode **56** einer zweiten Form zugeordnet ist. Die Extraktionsgitterelektroden **51, 53, 54** und die Fokussierungselektroden **52, 55, 56** sind in der Fig. 1, Fig. 2., Fig. 3 und in der Fig. 6 nicht eingezeichnet und werden anhand der Fig. 4 für das erste Ausführungsbeispiel und anhand Fig. 5 für das zweite Ausführungsbeispiel näher erläutert. Die Fig. 4 und die Fig. 5 sind nicht maßstabsgetreu. In Fig. 4 ist die Gitterspannung beispielhaft mit 0 bis +1kV angegeben. Abweichend hiervon kann beim Betrieb des Computertomographen **1** zum Beispiel eine Gitterspannung im Bereich von Null bis ±1kV anliegen.

In allen drei Ausführungsbeispielen des vorgeschlagenen Computertomographen **1** sind die Extraktionsgitterelektroden **51, 53, 54** bei einer Nichtinbetriebnahme geerdet oder für einen Betrieb galvanisch mit den Kathoden schaltbar, jedoch von allen anderen Komponenten der MBFEX-Röhre **20** galvanisch getrennt. Die Extraktionsgitterelektroden **51, 53, 54** sind wahlweise mit einem gleichförmig gepulsten positiven Potential von bis zu 1 kV beaufschlagbar.

In allen drei Ausführungsbeispielen sind die Fokussierungselektroden **52, 55, 56** bei einer Nichtinbetriebnahme ebenfalls geerdet, aber für einen Betrieb galvanisch mit der Anoden **6** schaltbar, jedoch ansonsten von allen anderen Komponenten der MBFEX-Röhre **20** galvanisch getrennt, wie dies repräsentativ und schematisch in Fig. 4 dargestellt ist. Die Fokussierungselektroden **52, 55, 56** sind wahlweise mit einem zeitlich konstanten negativen oder positiven Potential von bis zu 10 kV beaufschlagbar.

Für eine computergestützte Röntgenbildgebung mittels Tomosynthese liegt auf Anode **6** ein zeitlich konstantes Potential von 40 KV an, wobei zwischen der Anode **6** und der jeweils geschalteten Kathode **40, 41** ein gleichförmig gepulster elektrischer Gleichstrom von 30 mA fließt. Für eine computergestützte Röntgenbildgebung mittels HPEC-Tomosynthese dagegen liegt auf der betreffenden Anode ein zeitlich konstantes Potential von 120 kV an, wobei zwischen der Anode **6** und der jeweils geschalteten Kathode **40, 42** ein gleichförmig gepulster elektrischer Gleichstrom von 0.5 mA fließt.

In allen drei Ausführungsbeispielen des vorgeschlagenen Computertomographen **1** sind bei einer computergestützten Röntgenbildgebung mittels Tomosynthese eine Brennfleckgröße mit einem Durchmesser von 0.3 mm bis 0.6 mm und bei einer computergestützten Röntgenbildgebung mittels HPEC-Tomosynthese eine Brennfleckgröße mit einem Durchmesser von 0.1 mm realisierbar.

In allen drei Ausführungsbeispielen weist der vorgeschlagene Computertomograph **1** einen Stromregler, eine Gerätesteuerung, ein elektronisches Steuersystem (ECS = Electronic Control System), eine Kathoden-Hochspannungsquelle (CPS = Cathode Power Supply), eine Anoden-Hochspannungsquelle (APS = Anode Power Supply) und eine Gerätesteuerung auf. Die Anode **6** ist damit in eine strombasierte Stromregelung eingebunden, welche eine Messung des von den Kathoden **40, 42** emittierten Stroms zum Zweck der Regelung des Anodenstroms auf einen bestimmten Wert einschließt. Der Stromregler, die Gerätesteuerung, das elektronische Kontrollsystem, die Kathoden-Hochspannungsquelle, die Anoden-Hochspannungsquelle und die Gerätesteuerung sind Bestandteil einer elektronischen Regelungsvorrichtung. Der Stromregler, die Gerätesteuerung und das elektronische Steuersystem stellen ein elektronisches Leitsystem dar.

Die elektronische Regelungsvorrichtung weist einen elektrischen Hauptkreis und einen Regelkreis auf, wobei der Hauptkreis und der Regelkreis in einem Gleichstromkreis integriert sind. In dem Hauptkreis sind die Anoden-Hochspannungsquelle mit der Anode **6** und dem Stromregler, der Stromregler mit der Gerätesteuerung, die Gerätesteuerung mit dem elektronischen Steuersystem, das elektronische Steuersystem mit der Kathoden-Hochspannungsquelle und die Kathoden-Hochspannungsquelle in paralleler Schaltung mit den Kathoden **40, 41, 42** als auch mit der jeweiligen Gittervorrichtung **50** elektrisch verbunden. In dem Regelkreis ist die Anoden-Hochspannungsquelle über eine Rückführung mit dem Leitsystem elektrisch verknüpft. Hierbei ist das Leitsystem sowohl für die sequentiellen Schaltungen der Kathoden **40, 41, 42,** für die Regelung der Extraktionsgitterelektroden **51, 53, 54** und der Fokussierungselektroden **52, 55, 56** der jeweiligen Gittervorrichtung **50** als auch für die Regelung des Hauptkreisstroms vorgesehen, wobei auf den mit dem Leitsystem vorgegeben Hauptkreisstrom die elektrische Spannung der Kathoden-Hochspannungsquelle anpassbar ist.

Die MBFEX-Röhre **20** ist in allen drei Ausführungsbeispielen in Parallelrichtung **z** zu dem Flachbett-Röntgendetektor **30** verschiebbar. In allen Ausführungsbeispielen weist der Flachbett-Röntgendetektor **30** eine verschiebbare als auch öffnungsverstellbare Röntgenblende **31** auf, wobei mit der Röntgenblende **31** ein Abbildungsbereich **A** auf der Detektor-Oberfläche **D** des Flachbett-Röntgendetektor **30** auswählbar und verschiebbar ist.

In allen drei Ausführungsbeispielen des vorgeschlagenen Computertomographen ist die MBFEX-Röhre **20** bezüglich der Detektoroberfläche **D** in einer Senkrechtrichtung **y** abstandsverstellbar.

Bei einer Röntgenuntersuchung, beispielsweise einer weiblichen menschlichen Brust **70** als Untersuchungsobjekt, ist die Brust **70** zwischen der MBFEX-Röhre **20** und dem Röntgen-Flachbettdetektor **30** positioniert. In allen Ausführungsbeispielen des vorgeschlagenen Computertomographen **1** ist die Brust **70** einer Patientin auf einer Platte **32** über der Röntgenblende **31** aufgelegt, wobei die Platte **32** durchlässig für Röntgenstrahlung ist. Mit einer Kompressionsscheibe **33** ist die Brust **70** auf der Platte **32** temporär für die Röntgenuntersuchung fixiert.

Das erste Ausführungsbeispiel des vorgeschlagenen Computertomographen **1** wird nachfolgend anhand der Fig. 1, der Fig. 2, der Fig. 3 und der Fig. 4 näher erläutert.

Die Fig. 1 zeigt eine schematische Ansicht der MBFEX-Röhre **20** des ersten Ausführungsbeispiels eines Computertomographen **1.** Die Fig. 1 ist nicht maßstabsgetreu. Die Vakuumröhre **21,** das Röntgenfenster **22** und der Kollimator **23** der MBFEX-Röhre **20,** die Gittervorrichtung **50** als auch die Röntgenblende **31** sind in der Fig. 1 nicht sichtbar. In der Fig. 1 sind Röntgenstrahlenbündel **b** in Form von Fächerbündeln **f** eingezeichnet, welche sequentiell erzeugbar sind. Die Röntgenstrahlenbündel **b** sind in ihrer Röntgen-Hauptemissionsrichtung **h** auf die einliegende Brust **70** ausgerichtet.

Die Fig. 2 zeigt den vorgeschlagenen Computertomographen **1** in seinem ersten Ausführungsbeispiel in einer Seitenansicht. In der Fig. 2 ist der Computertomograph **1** während einer computergestützten Röntgenbildgebung der gesamten Brust **70** mittels Tomosynthese schematisiert, bei welcher alle Röntgenstrahlenbündel **b** mit dem Kollimator **23** in Form von Kegelbündeln **c** eingestellt und die MBFEX-Röhre **20** ortsfest gehalten sind. Der Abbildungsbereich **A** ist mit der Röntgenblende **31** so eingestellt, dass dieser die Brust **70** gerade vollständig umfasst.

Die Fig. 3 zeigt den vorgeschlagenen Computertomographen **1** in seinem ersten Ausführungsbeispiel ebenfalls in einer Seitenansicht. In der Fig. 3 ist der Computertomograph **1** während einer computergestützten Röntgenbildgebung einer ROI **71** der Brust **70** mittels HPEC-Tomosynthese schematisiert, bei welcher alle Röntgenstrahlenbündel **b** mit dem Kollimator **23** in Form von Fächerbündeln **f** eingestellt sind, wobei die jeweiligen Röntgen-Fächerebenen **F** senkrecht zu der Parallelrichtung **z** sind. Bei der Röntgenbildaufnahme der ROI **71** ist der Abbildungsbereich **A** synchron mit der MBFEX-Röhre **20** in der Parallelrichtung **z** verschiebbar. Der Röntgen-Auftreffbereich **B** deckt den Abbildungsbereich **A** vollständig ab und ist rechteckig. Die Röntgen-Fächerebene **F** ist hierbei parallel zur Längsseite des Abbildungsbereiches **A** und halbiert den Abbildungsbereich **A** geometrisch. Die MBFEX-Röhre **20** ist bei der Röntgenbildaufnahme derer ROI **71** synchron mit der Röntgenblende **31** angesteuert. Für jeden Verschiebungsschritt der MBFEX-Röhre **20** und der Röntgenblende **31** in dem für die ROI **71** gewählten Abbildungsbereich **A** ist eine Sequenz von Schaltungen der Kathoden **40** und damit eine Röntgenbildaufnahme bewerkstelligt.

Mit vorgeschlagenen Computertomographen **1** in seiner ersten Ausführungsform sind mit lediglich einer Anordnung von Kathoden **40** einer Sorte jeweils Röntgenbildaufnahmen für zwei unterschiedliche computergestützte Verfahren zur Erzeugung von Querschnittsansichten und Volumenstrukturen des untersuchten Objektes bewerkstelligbar. Hierzu sind für eine computergestützte Röntgenbildgebung mittels HPEC-Tomosynthese die Fokussierungselektroden **52** mit einem höheren negativen elektrischen Potential als für eine computergestützte Röntgenbildgebung mittels Tomosynthese beaufschlagt.

Die Fig. 4 zeigt eine Teilansicht der Gittervorrichtung **50** des ersten Ausführungsbeispiels des vorgeschlagenen Computertomographen **1,** welche auf die exemplarische Darstellung einer Kathode **40** mit der zu dieser zugeordneten Fokussierungselektrode **52** beschränkt ist. In der Fig. 4 ist links ist eine Teilansicht der Gittervorrichtung **50** bezüglich der Längsrichtung der Kathoden **40** und rechts eine Teilansicht der Gittervorrichtung **50** bezüglich der Querrichtung der Kathoden **40** dargestellt; in beiden Teilansichten ist die Extraktionsgitterelektrode **51** in jeweils geschnittener Ansicht dargestellt. Die Gittervorrichtung **50** und die Kathoden **40** sind auf einem gemeinsamen keramischen Träger **80** angeordnet. Die Extraktionsgitterelektrode **51** und die Kathoden **40** sind jeweils über eine Metallschicht **81** mit dem keramischen Träger **80** verbunden. Extraktionsgitterelektrode **51** ist aus Wolfram gefertigt. Die Metallschicht **81** ist für die elektrische Kontaktgebung der Kathoden **40** und der Extraktionsgitterelektrode **51** vorgesehen, über welche die Kathoden **40** und die Extraktionsgitterelektrode **51** mit dem elektronischen Kontrollsystem elektrisch verbunden sind. Das elektronische Kontrollsystem ist in der Fig. 4 schematisch eingezeichnet. In der Fig. 4 ist eine Kathode **40** während ihrer elektronischen Ansteuerung zusammen mit der zugeordneten Fokussierungselektrode **52** im eingeschalteten Zustand dargestellt, wobei die Extraktionsgitterelektrode **51** ebenfalls eingeschaltet und der Feldlinienverlauf des erzeugten Elektronenstrahlenbündel **a** schematisch eingezeichnet ist.

Das zweite Ausführungsbeispiel des vorgeschlagenen Computertomographen **1** wird nachfolgend anhand der Fig. 5 erläutert. Die Fig. 5 zeigt ebenfalls eine Teilansicht der Gittervorrichtung **50,** welche auf die exemplarische Darstellung zweier Kathoden **41, 42** mit den jeweils zu diesen zugeordneten Fokussierungselektrode **55, 56** beschränkt ist. In der Fig. 5 ist oben ist eine Teilansicht der Gittervorrichtung **50** bezüglich der Längsrichtung der Kathoden **41, 42** und unten eine Teilansicht der Gittervorrichtung **50** bezüglich der Querrichtung der Kathoden **41, 42** dargestellt; in beiden Teilansichten sind die Extraktionsgitterelektroden **53, 54** ebenfalls in jeweils geschnittener Ansicht dargestellt. Die Kathoden **41** der ersten Sorte weisen eine kleinere Fläche als die Kathoden **42** der zweiten Sorte auf. Bei einer Röntgenbildaufnahme sind entweder die Kathoden **41** der ersten Sorte oder die Kathoden **42** der zweiten Sorte sequentiell angesteuert, wobei die Kathoden **41** der ersten Form für eine computergestützte Röntgenbildgebung mittels HPEC-Tomosynthese und die Kathoden **42** der zweiten Form für eine computergestützte Röntgenbildgebung mittels Tomosynthese vorgesehen sind. In der Fig. 5 ist eine Kathode **41** während ihrer elektronischen Ansteuerung zusammen mit der zugeordneten Extraktionsgitterelektrode **53** und zugeordneten Fokussierungselektrode **55** im eingeschalteten Zustand während einer computergestütztenm Röntgenbildgebung mittels HPEC-Tomosynthese dargestellt.

Das dritte Ausführungsbeispiel des vorgeschlagenen Computertomographen **1** wird nachfolgend anhand der Fig. 6 erläutert. Die Fig. 6 ist auf die exemplarische Darstellung von insgesamt acht Kathoden **41, 42** der MBFEX-Röhre **20** beschränkt. Die Gittervorrichtung **50** ist in der Fig. 6 nicht sichtbar.

In allen drei Ausführungsbeispielen des vorgeschlagenen Computertomographen **1** sind mit der bogenförmigen Anode **6** tote Winkel um die Parallelrichtung **z** vermeidbar. Somit ist in allen Ausführungsbeispielen jede ROI **71** um die Parallelrichtung **z** vollständig, gleichförmig und mit hoher Auflösung bei gleichzeitiger verhältnismäßig geringer Röntgenstrahlexposition des Untersuchungsobjektes abbildbar, wie dies an den eingezeichneten Röntgenstrahlenbündel **b** exemplarisch in Fig. 1, Fig. 2 und Fig. 3 zeichnerisch verdeutlicht ist. Der vorgeschlagene Computertomograph **1** in allen drei Ausführungsbeispielen, insbesondere die MBFEX-Röhre **20,** zeichnet sich durch eine besonders kompakte Bauform aus.

### Bezugszeichenliste

- 1: Computertomograph

- 20: MBFEX-Röhre
- 21: Vakuumröhre
- 22: Röntgenfenster
- 23: Kollimator

- 30: Flachbett-Röntgendetektor
- 31: Röntgenblende
- 32: Platte
- 33: Kompressionsscheibe

- 40: Kathode
- 41: Kathode einer ersten Sorte
- 42: Kathode einer zweiten Sorte

- 50: Gittervorrichtung
- 51: Extraktionsgitterelektrode
- 52: Fokussierungselektrode
- 53: Extraktionsgitterelektrode einer ersten Form
- 54: Extraktionsgitterelektrode einer zweiten Form
- 55: Fokussierungselektrode einer ersten Form
- 56: Fokussierungselektrode einer zweiten Form

- 6: Anode

- 70: Brust
- 71: ROI

- 80: keramischer Träger
- 81: Metallschicht
- D: Detektor-Oberfläche
- b: Röntgenstrahlenbündel
- h: Röntgen-Hauptemissionsrichtung
- c: Röntgen-Kegelbündel
- f: Röntgen-Fächerbündel
- B: Röntgen-Auftreffbereich
- F: Röntgen-Fächerebene
- Q: Röntgenstrahlenquelle
- a: Elektronenstrahlenbündel
- z: Parallelrichtung
- A: Abbildungsbereich
- y: Senkrechtrichtung

## Patentansprüche

1. Computertomograph (1) für die mammographische Röntgenbildgebung, welcher eine MBFEX-Röhre (20) und einen Flachbett-Röntgendetektor (30) aufweist, wobei in der MBFEX-Röhre (20) eine Mehrzahl von Kathoden (40) reihenförmig fest angeordnet sind, die Kathoden (40) für die Feldemission von Elektronen vorgesehen sind, sowohl die Geometrie als auch die Strahlungsdichte als auch der Wellenlängenbereich eines Röntgenstrahlenbündels (b) einstellbar ist, die MBFEX-Röhre (20) in Parallelrichtung (z) zu dem Flachbett-Röntgendetektor (30) verschiebbar ist, der Flachbett-Röntgendetektor (30) eine verschiebbare als auch öffnungsverstellbare Röntgenblende (31) aufweist und mit der Röntgenblende (31) ein Abbildungsbereich (A) auf der Detektor-Oberfläche (D) des Flachbett-Röntgendetektor (30) auswählbar und verschiebbar ist.

2. Computertomograph (1) nach Anspruch 1, **dadurch gekennzeichnet, dass** die Kathoden (40) Kohlenstoffnanoröhren enthalten.

3. Computertomograph (1) nach Anspruch 1 oder 2, **dadurch gekennzeichnet, dass** die Kathoden (40) zur Emission von Elektronen ausgebildete Nanostäbchen enthalten, welche mindestens einen Stoff enthalten, der aus einer Gruppe an Stoffen ausgewählt ist, die Metalloxide, Metallsulfide, Nitride, Carbide und Silizium enthält.

4. Computertomograph (1) nach einem der Ansprüche 1 bis 3, **dadurch gekennzeichnet, dass** die MBFEX-Röhre (20) eine fest angeordnete Gittervorrichtung (50) aufweist, wobei in der Gittervorrichtung (50) eine allen Kathoden (40) gemeinsame Extraktionsgitterelektrode (51) oder jeder einzelnen Kathode (40) jeweils eine separate Extraktionsgitterelektrode (51) zugeordnet ist, jede Extraktionsgitterelektrode (51) direkt über den Kathoden (40) angeordnet und für die Feldextraktion von Elektronen vorgesehen ist.

5. Computertomograph (1) nach Anspruch 4, **dadurch gekennzeichnet, dass** die Gittervorrichtung (50) direkt über jeder Extraktionsgitterelektrode (51) für jede einzelne Kathode (40) jeweils eine separat zugeordnete Fokussierungselektrode (52) aufweist, wobei jede Fokussierungselektrode (52) für die Fokussierung der extrahierten Elektronen als Elektronenstrahlenbündel (a) vorgesehen ist.

6. Computertomograph (1) nach einem der Ansprüche 1 bis 5, **dadurch gekennzeichnet, dass** die MBFX-Röhre (20) mehr als eine reihenförmige Anordnung von gleichen Kathoden (40) oder von Kathoden (41, 42) verschiedener Sorten aufweist, wobei jeweils eine reihenförmige Anordnung nicht mehr als eine Sorte von Kathoden (41, 42) umfasst.

7. Computertomograph (1) nach Anspruch 6, **dadurch gekennzeichnet, dass** sich die Kathoden (41, 42) verschiedener Sorten zumindest hinsichtlich ihrer Fläche, welche sie auf einem gemeinsamen Träger (80) einnehmen, voneinander unterscheiden.

8. Computertomograph (1) nach den Ansprüchen 4, 5, 6 und 7, **dadurch gekennzeichnet, dass** jeder reihenförmigen Anordnung von Kathoden (40, 41, 42) Extraktionsgitterelektroden (53, 54) und Fokussierungselektroden (55, 56) jeweils eines Typs zugeordnet sind, wobei sich die reihenförmigen Anordnungen von Kathoden (40, 41, 42) durch mindestens einen Typ von Extraktionsgitterelektroden (53, 54) und / oder mindestens einen Typ von Fokussierungsgitterelektroden (55, 56) voneinander unterscheiden.

9. Computertomograph (1) nach einem der Ansprüche 1 bis 8, **dadurch gekennzeichnet, dass** mehrere Kathoden (40, 41, 42) auf eine gemeinsame feste Anode (6) ausgerichtet sind.

10. Computertomograph (1) nach Anspruch 9, **dadurch gekennzeichnet, dass** die die Anode (6) bogenförmig ausgebildet und konkav auf die Detektoroberfläche (D) ausgerichtet ist.

11. Computertomograph (1) nach Anspruch 9 oder 10, **dadurch gekennzeichnet, dass** die Anode (6) als flüssigkeitsgekühlte Anode ausgebildet ist.

12. Computertomograph (1) nach einem der Ansprüche 1 bis 11, **dadurch gekennzeichnet, dass** mit einem Kollimator (23) das Röntgenstrahlenbündel (b) wahlweise als ein Röntgen-Kegelbündel (c) mit einem kreisförmigen oder ellipsenförmigen Röntgen-Auftreffbereich (B) oder als ein Röntgen-Fächerbündel (f) mit einem linienförmigen Röntgen-Auftreffbereich (B) einstellbar ist, wobei das Röntgen-Fächerbündel (f) eine den Röntgen-Auftreffbereich (B) längsseitig halbierende Röntgen-Fächerebene (F) aufweist.

13. Computertomograph (1) nach Anspruch 12, **dadurch gekennzeichnet, dass** der Abbildungsbereich (A) synchron mit der MBFEX-Röhre (20) in Parallelrichtung (z) verschiebbar und das Röntgenstrahlenbündel (b) als Röntgen-Fächerbündel (f) einstellbar ist, wobei die Röntgen-Fächerebene (F) senkrecht zu der Parallelrichtung (z) ist, der Röntgen-Auftreffbereich (B) den Abbildungsbereich (A) vollständig abdeckt, der Abbildungsbereich (A) rechteckig ist, die Röntgen-Fächerebene (F) parallel zur Längsseite des Abbildungsbereiches (A) ist, die Röntgen-Fächerebene (F) den Abbildungsbereich (A) geometrisch halbiert und die MBFEX-Röhre (20) synchron mit der Röntgenblende (31) ansteuerbar ist.

14. Computertomograph (1) nach einem der Ansprüche 1 bis 13, **dadurch gekennzeichnet, dass** die MBFEX-Röhre (20) in einer Senkrechtrichtung (y) abstandsverstellbar bezüglich der Detektoroberfläche (D) ist.

15. Computertomograph (1) nach einem der Ansprüche 1 bis 14, **dadurch gekennzeichnet, dass** die Anode (6) Teil eines zur strombasierten Stromregelung ausgebildeten Regelkreises, welcher eine Messung des von den Kathoden (40, 41, 42) emittierten Elektronenstroms einschließt, ist.

## Claims

1. A computed tomography system (1) for mammographic X-ray imaging which comprises an MBFEX tube (20) and a flat-bed X-ray detector (30), wherein a plurality of cathodes (40) is fixedly arranged in rows in the MBFEX tube (20), the cathodes (40) are provided for the field emission of electrons, both the geometry and the radiation density and the wavelength range of an X-ray beam (b) is adjustable, the MBFEX tube (20) is movable in a parallel direction (z) to the flat-bed X-ray detector (30), the flat-bed X-ray detector (30) comprises a movable and also opening-adjustable X-ray aperture (31) and an imaging area (A) on the detector surface (D) of the flat-bed X-ray detector (30) can be selected and moved using the X-ray aperture (31).

2. The computed tomography system (1) according to claim 1, **characterized in that** the cathodes (40) contain carbon nanotubes.

3. The computed tomography system (1) according to claim 1 or 2, **characterized in that** the cathodes (40) contain nanorods designed for the emission of electrons, said nanorods containing at least one substance which is selected from a group of substances containing metal oxides, metal sulphides, nitrides, carbides and silicon.

4. The computed tomography system (1) according to one of claims 1 to 3, **characterized in that** the MBFEX tubes (20) have a fixedly arranged grid device (50), wherein in the grid device (50) a shared extraction grid electrode (51) is assigned to all cathodes (40) or a separate extraction grid electrode (51) is assigned to each individual cathode (40) in each case, each extraction grid electrode (51) is arranged directly above the cathodes (40) and is provided for the field extraction of electrons.

5. The computed tomography system (1) according to claim 4, **characterized in that** the grid device (50) has a separately assigned focussing electrode (52) above each extraction grid electrode (51) for each individual cathode (40), wherein each focussing electrode (52) is provided for the focussing of the extracted electrons as an electron beam (a).

6. The computed tomography system (1) according to one of claims 1 to 5, **characterized in that** the MBFX tube (20) comprises more than one arrangement in rows of identical cathodes (40) or of cathodes (41, 42) of different kinds, wherein each arrangement in rows comprises no more than one kind of cathodes (41, 42) in each case.

7. The computed tomography system (1) according to claim 6, **characterized in that** the cathodes (41, 42) of different kinds differ from one another at least in terms of their surface which they cover on a shared carrier (80).

8. The computed tomography system (1) according to claims 4, 5, 6 and 7, **characterized in that** each arrangement of cathodes (40, 41, 42) in rows is assigned extraction grid electrodes (53, 54) and focussing electrodes (55, 56) of one type in each case, wherein the arrangements of cathodes (40, 41, 42) in rows are distinguished from one another by at least one type of extraction grid electrodes (53, 54) and/or at least one type of focussing grid electrodes (55, 56).

9. The computed tomography system (1) according to one of claims 1 to 8, **characterized in that** multiple cathodes (40, 41, 42) are aimed at a common fixed anode (6).

10. The computed tomography system (1) according to claim 9, **characterized in that** the anode (6) has an arcuate design and is aimed at the detector surface (D) in a concave manner.

11. The computed tomography system (1) according to claim 9 or 10, **characterized in that** the anode (6) is designed as a liquid-cooled anode.

12. The computed tomography system (1) according to one of claims 1 to 11, **characterized in that** using a collimator (23) the X-ray beam (b) can be optionally set either as an X-ray cone beam (c) having a circular or elliptical X-ray impact region (B) or as an X-ray fan beam (f) having a linear X-ray impact region (B), wherein the X-ray fan beam (f) has an X-ray fan plane (F) which halves the X-ray impact region (B) on the longitudinal side.

13. The computed tomography system (1) according to claim 12, **characterized in that** the imaging region (A) can be moved synchronously with the MBFEX tube (20) in a parallel direction (z) and the X-ray beam (b) can be set as an X-ray fan beam (f), wherein the X-ray fan plane (F) is perpendicular to the parallel direction (z), the X-ray impact region (B) completely covers the imaging region (A), the imaging region (A) is rectangular, the X-ray fan plane (F) is parallel to the longitudinal side of the imaging region (A), the X-ray fan plane (F) halves the imaging region (A) geometrically and the MBFEX tube (20) can be controlled synchronously with the X-ray aperture (31).

14. The computed tomography system (1) according to one of claims 1 to 13, **characterized in that** the MBFEX tube (20) is distance-adjustable in a vertical direction (y) in respect of the detector surface (D).

15. The computed tomography system (1) according to one of claims 1 to 14, **characterized in that** the anode (6) is part of a control circuit designed for the current-based current regulation control which includes a measurement of the electron flow emitted by the cathodes (40, 41, 42).

## Revendications

1. Tomodensitomètre (1) pour l'imagerie par rayons X mammographique, lequel présente un tube MBFEX (20) et un détecteur à rayons X à plat (30), dans lequel, dans le tube MBFEX (20), une pluralité de cathodes (40) sont agencées à demeure en rangée, les cathodes (40) sont prévues pour l'émission de champs d'électrons, la géométrie tout comme également la densité de rayonnement qu'également la plage des longueurs d'onde d'un faisceau de rayons X (b) sont réglables, le tube MBFEX (20) est déplaçable en direction parallèle (z) au détecteur à rayons X à plat (30), le détecteur à rayons X à plat (30) présente un diaphragme pour rayons X (31) déplaçable tout comme également à ouverture réglable, et avec le diaphragme pour rayons X (31), une zone de représentation (A) sur la surface de détecteur (D) du détecteur à rayons X à plat (30) peut être sélectionnée et déplacée.

2. Tomodensitomètre (1) selon la revendication 1, **caractérisé en ce que** les cathodes (40) contiennent des nanotubes de carbone.

3. Tomodensitomètre (1) selon la revendication 1 ou 2, **caractérisé en ce que** les cathodes (40) comprennent des nanotiges réalisées pour l'émission d'électrons, lesquelles contiennent au moins une substance sélectionnée dans le groupe comprenant des oxydes métalliques, des sulfures métalliques, des nitrures, des carbures, et du silicium.

4. Tomodensitomètre (1) selon l'une des revendications 1 à 3, **caractérisé en ce que** le tube MBFEX (20) présente un dispositif de grille (50) agencé à demeure, dans lequel, dans le dispositif de grille (50), on associe une électrode à grille d'extraction (51) commune à toutes les cathodes (40) ou respectivement une électrode à grille d'extraction séparée (51) à chaque cathode individuelle (40), chaque électrode à grille d'extraction (51) étant disposée directement au-dessus des cathodes (40) et étant prévue pour l'extraction de champ d'électrons.

5. Tomodensitomètre (1) selon la revendication 4, **caractérisé en ce que** le dispositif de grille (50) directement au-dessus de chaque électrode à grille d'extraction (51) présente, pour chaque cathode (40) individuelle, respectivement une électrode de concentration (52) associée séparément, dans lequel chaque électrode de concentration (52) est prévue pour la concentration des électrons extraits en tant que faisceau d'électrons (a).

6. Tomodensitomètre (1) selon l'une des revendications 1 à 5, **caractérisé en ce que** le tube MBFX (20) présente plus d'un agencement en forme de rangée de mêmes cathodes (40) ou de différentes sortes de cathodes (41, 42), dans lequel respectivement un agencement en forme de rangée ne comprend pas plus d'une sorte de cathodes (41, 42).

7. Tomodensitomètre (1) selon la revendication 6, **caractérisé en ce que** les cathodes (41, 42) de différentes sortes diffèrent entre elles au moins concernant leur surface qu'elles occupent sur un support (80) commun.

8. Tomodensitomètre (1) selon les revendications 4, 5, 6 et 7, **caractérisé en ce que** l'on associe, à chaque agencement en forme de rangée de cathodes (40, 41, 42), des électrodes à grille d'extraction (53, 54) et des électrodes de concentration (55, 56) d'un type respectif, dans lequel les agencements en forme de rangée de cathodes (40, 41, 42) diffèrent entre eux de par au moins un type d'électrodes à grille d'extraction (53, 54) et/ou au moins un type d'électrodes de concentration (55, 56).

9. Tomodensitomètre (1) selon l'une des revendications 1 à 8, **caractérisé en ce que** plusieurs cathodes (40, 41, 42) sont orientées vers une anode fixe commune (6).

10. Tomodensitomètre (1) selon la revendication 9, **caractérisé en ce que** l'anode (6) est réalisée en forme d'arc et est orientée de manière concave vers la surface du détecteur (D).

11. Tomodensitomètre (1) selon la revendication 9 ou 10, **caractérisé en ce que** l'anode (6) est réalisée en tant qu'anode refroidie par liquide.

12. Tomodensitomètre (1) selon l'une des revendications 1 à 11, **caractérisé en ce qu'**avec un collimateur (23), le faisceau de rayons X (b) est réglable au choix en tant que faisceau conique de rayons X (c) avec une zone d'incidence des rayons X circulaire ou elliptique ou en tant que faisceau en éventail de rayons X (f) avec une zone d'incidence des rayons X linéaire (B), dans lequel le faisceau en éventail de rayons X (f) présente un plan de radiographie en éventail (F) coupant longitudinalement en deux la zone d'incidence des rayons X (B).

13. Tomodensitomètre (1) selon la revendication 12, **caractérisé en ce que** la zone de représentation (A) est déplaçable de manière synchrone avec le tube MBFEX (20) en direction parallèle (z) et que le faisceau de rayons X (b) est réglable en tant que faisceau en éventail de rayons X (f), dans lequel le plan de radiographie en éventail (F) est perpendiculaire à la direction parallèle (z), la zone d'incidence des rayons X (B) recouvre complètement la zone de représentation (A), la zone de représentation (A) est rectangulaire, le plan de radiographie en éventail (F) est parallèle au côté longitudinal de la zone de représentation (A), le plan de radiographie en éventail (F) coupe la zone de représentation (A) en deux de façon géométrique et le tube MBFEX (20) peut être commandé de manière synchrone avec le diaphragme pour rayons X (31).

14. Tomodensitomètre (1) selon l'une des revendications 1 à 13, **caractérisé en ce que** le tube MBFEX (20) est réglable concernant la distance dans une direction perpendiculaire (y) par rapport à la surface du détecteur (D).

15. Tomodensitomètre (1) selon l'une des revendications 1 à 14, **caractérisé en ce que** l'anode (6) est une partie d'un circuit de régulation réalisé pour la régulation du courant par flux, lequel comprend une mesure du flux d'électrons émis par les cathodes (40, 41, 42).
